# EUROPEAN PATENT SPECIFICATION

(11) **EP 1 761 257 B1**
(45) Date of publication and mention of the grant of the patent: **31.12.2014**
(21) Application number: 05730316.6
(22) Date of filing: 05.04.2005
(51) Int. Cl.: A61K 31/22, A61P 3/00

(54) **USE OF ACETYL L-CARNITINE FOR THE PREPARATION OF A MEDICINE FOR THE TREATMENT OF NEUROPATHIC PAIN IN DIABETIC PATIENTS**
VERWENDUNG VON ACETYL L-CARNITIN ZUR HERSTELLUNG EINES MEDIKAMENTS ZUR BEHANDLUNG VON NEUROPATHISCHEN SCHMERZEN VON DIABETIKERN
UTILISATION D'ACETYL L-CARNITINE DANS LA PREPARATION D'UN MEDICAMENT DESTINE AU TRAITEMENT DE DOULEURS NEUROPATHIQUES CHEZ DES PATIENTS DIABETIQUES

(30) Priority: 01.07.2004 IT RM20040327
(43) Date of publication of application: 14.03.2007
(73) Proprietor: SIGMA-TAU Industrie Farmaceutiche Riunite S.p.A., 00144 Roma (IT)
(72) Inventor: CALVANI, Menotti, Via Pontina KM 30,400, 00040 Pomezia (IT); AMATO, Antonino, Via Pontina KM. 30,400, 00040 Pomezia (IT)
(86) International application number: PCT/EP2005/003557
(87) International publication number: WO 2006/002698

(56) References cited:
- US-A- 4 751 242
- GRANDIS ET AL: "Acetyl-L-Carnitine (Levacecarnine) in the Treatment of Diabetic Neuropathy" DRUGS R&D, vol. 3, no. 4, 2002, - 2002 pages 223-231, XP001206970
- GRANDIS: "Tolerability and efficacy of L Acetylcarnitine in Patients with Peiferal neuropathies" CLIN.DRUG.INVEST, vol. 15, no. 2, 1998, - 1998 pages 73-79, XP001106152

## Description

The invention described herein relates to the use of acetyl L-carnitine for the preparation of a medicine for treatment of neuropathic pain in patients with type 2 diabetes not on insulin treatment.

Diabetic neuropathy is the most frequent peripheral neuropathy in the western world and includes different forms of neuropathy, among which diabetic polyneuropathy is the most common. The anatomico-pathological picture of diabetic neuropathy consists in a focal or diffuse aspecific loss of fibres, with demyelination associated with structural or intraneuronal abnormalities of the connective tissue or of the small vessels.

The causes of diabetic neuropathy are not known. Metabolic disequilibrium secondary to hyperglycaemia and ischaemia of the "vasa nervorum" are the best known pathogenetic mechanisms.

Various metabolic abnormalities and biochemical modifications have been documented both in experimental models of diabetes and in diabetic patients, including an increase in glucose metabolism and a reduction in myoinositol.

The characteristic symptoms of diabetic polyneuropathy consist in the presence of lancinating or burning pain accompanied by clinical signs of symmetrical impairment of sensitivity, motility and/or deep tendon reflexes; these symptoms are predominant in the distal segments of the lower limbs.

To date the therapeutic approach adopted by investigators to the use of acetyl L-carnitine for the treatment of neuropathic pain in patients with diabetes has failed to take account of the selection of patients on the basis of the type of diabetes and the type of antidiabetic agents taken up to the time of entry into clinical trials.

In fact, in the Journal of the American Diabetes Association June 2002, Vol. 51, Suppl., a multicentre clinical trial to assess the effect of acetyl L-carnitine treatment on neuropathy patients with type I and type 2 diabetes is described.

The results obtained in this trial are contradictory, showing improvements in one group but not in the other. At the end of the trial, the authors report that in the patient group recruited in Europe there was no improvement in pain symptoms, whereas an improvement was registered in the American and Canadian group.

The authors suggest that greater improvements were obtained in the group of type 2 diabetic patients with obesity.

In this study, too, the authors (two of whom are the inventors in the present patent application) failed to understand at the end of the trial they had conducted that a different selection of the patients to be recruited would have led to different, better and less variable results.

Moreover, a different patient selection would have avoided non-responders being included in the trial and treated with a compound which was of no use to them, i.e. ineffective, thus subjecting these patients to the risk of incurring side effects without receiving any therapeutic benefit.

In this case, thanks to the well-known lack of severe side effects of the study compound used, the non-responders were not harmed by the drug, but the same could not be said if the study compound had been another drug, associated with more serious side effects.

Thus, in this trial, the patients were not selected on the basis of the type of therapy they had received to control their hyperglycaemia and therefore it was not understood what the cause of the improvement in one group and the lack of improvement in the other group were due to.

A report published in "Giornale Italiano di Diabetologia, 1998, Vol.18, pp. 30-31, abs" describes the effect of acetyl L-carnitine treatment on NIDDM diabetic patients, suffering from sensorimotor polyneuropathy.

The authors report that the results obtained in this study show an improvement in symptoms.

In this study, too, the patients were not selected on the basis of the type of therapy they had received to control their hyperglycaemia up to the time of entry into the clinical trial.. Therefore, in this case, too, it is impossible to establish whether or not a different patient selection might have led to different experimental results.

In fact, the authors suggest that only further clinical trials of longer duration would be able to demonstrate the efficacy of acetyl L-carnitine in diabetic peripheral neuropathy.

As will be explained more clearly here below, the authors were wrong: it was not by increasing the number of patients recruited into the clinical trial that the investigators would have been able to understand whether or not the study compound was active in the treatment of diabetic peripheral neuropathy; these objectives would have been achievable only through a different selection of the patients to be included in the clinical trial.

A report published in Drugs in Research and Development 2002, Vol. 3 (4), pp. 223-31, 2002 describes a clinical trial in which type I and II diabetics with symmetrical distal polyneuropathy or multineuropathy were recruited.

The results obtained in this clinical trial show that 39% of patients treated with acetyl L-carnitine out of 333 recruited presented an improvement in pain symptoms.

In this study, too, the patients were not selected on the basis of the type of therapy they had received to control their hyperglycaemia, and therefore in this case, too, it was impossible to understand what the cause of the improvement was or in what particular patient subgroup this compound is active and in what patient subgroup it is ineffective.

A report published in Diabetologia 1995, Vol/ISS/ p. 38/1 (123) describes the effect of treatment with acetyl L-carnitine on diabetic patients with diabetic peripheral neuropathy treated with insulin or with oral antidiabetic agents.

The results obtained in this trial show an improvement in the symptoms.

In this study, too, the patients were not selected on the basis of the type of diabetes or the type of therapy they had received to control their hyperglycaemia, and therefore in this case, too, it was impossible to understand what the cause of the improvement was or in what particular patient subgroup this compound is active and in what patient subgroup it is ineffective.

In fact, the authors suggest that only further clinical trials of longer duration would be able to demonstrate the efficacy of acetyl L-carnitine in diabetic peripheral neuropathy.

As already mentioned, the authors were wrong: it was not by increasing the number of patients recruited into the clinical trial that the investigators would have been able to understand whether or not the study compound was active in the treatment of diabetic peripheral neuropathy; these objectives would have been achievable only through a different selection of the patients to be included in the clinical trial.

In an article in *"Il giornale dei congressi medici,* 5, 14-19,1993" the author states that in a clinical trial 120 patients with diabetic neuropathy showed a distinct improvement in symptoms after treatment with acetyl L-carnitine.

The author of this article is one of the inventors in this patent application, and at the time he was commenting on, or became aware of, this clinical trial he had not understood that a different selection of the patients to be recruited would have led to better results than those obtained.

In fact, the author does not mention whether the patients were suffering from type I or type 2 diabetes.

In this case, too, better patient selection would have made it possible to exclude non-responders, which would have avoided them having to take a drug which for them was useless, ineffective and potentially damaging. As already mentioned, thanks to the well-known lack of severe side effects of the study compound, the non-responders were not harmed by taking the drug, but the same could not be said if the study compound was another drug, associated with more serious side effects.

Thus, in this study, too, the patients were not selected on the basis of the type of diabetes they were suffering from and therefore it was impossible to understand what subgroup of patients obtained improvement from treatment with acetyl L-carnitine.

In the abstracts of the National Congress of the Italian Society of Nephrophysiology, Perugia 1-4 June, 1994, p. 98, one report describes the effect of treatment with acetyl L-carnitine on diabetic patients treated with insulin or with oral antidiabetic agents, suffering from diabetic peripheral neuropathy.

The results obtained in this study show an improvement in symptoms, and the authors suggest that the improvement in the pain component in the treated group should be considered with great caution, amongst other things owing to the fact that it cannot be "weighed" precisely and they go so far as to advance complicated hypotheses of activity at nerve fibre level.

In this study, too, the patients were not selected on the basis of the type of diabetes they were suffering from and the type of therapy they had received to control their hyperglycaemia and therefore in this case, too, it was impossible to understand what subgroup of patients obtained improvement.

A report in Clin. Drug. Invest, Vol. 10 (6), pp. 317-22 1995, describes the effect of acetyl L-carnitine treatment on 426 patients with peripheral neuropathy of different origins, including 56 patients with diabetic neuropathy (13.1% of the entire study population). No information is provided regarding the type of diabetes or the type of treatment the patients had received to control their hyperglycaemia.

The results obtained in this study show an improvement in pain symptoms and the authors suggest that further studies in individual well-matched subgroups of neuropathic patients should be carried out because in their experimental model the groups were not large enough, and the treatment period not long enough for analysis to be undertaken.

As already mentioned, in this study, too, the authors were wrong: it was not by increasing the number of patients recruited into the clinical trial that the investigators would have been able to understand whether or not the study compound was active in the treatment of diabetic peripheral neuropathy.

Thus, in this study, too, the patients were not selected on the basis of the type of diabetes they were suffering from and the type of therapy they had received to control their hyperglycaemia and therefore in this case, too, it was impossible to understand what the cause of the improvement was and in what subgroup of patients it occurred.

A report in the Journal of the Neurological Sciences, 1997, Supp. to Vol. 150, describes the effect of acetyl L-carnitine treatment on patients with polyneuropathy.

In this study no information is provided regarding the type of diabetes and the type of treatment the patients had received to control their hyperglycaemia.

The results obtained in this study show an improvement in objective signs of peripheral neuropathy and in the nerve conduction velocity.

Thus, in this study, too, the patients were not selected on the basis of the type of diabetes they were suffering from and the type of therapy they had received to control their hyperglycaemia and therefore in this case, too, it was impossible to understand which specific subgroup was the responder group.

In the abstracts of the IX National Congress of the Italian Society of Clinical Pharmacology - II Congress of the Mediterranean Society of Clinical Pharmacology "Therapeutic Advances and New Health Problems", Venice, 8-10/ 10/ 1991 ABS one report describes the effect of acetyl L-carnitine treatment on 500 patients with peripheral neuropathy of different origins. No information is provided regarding the type of diabetes or the type of treatment the diabetic patients had received to control their hyperglycaemia.

The results obtained in this study show an improvement in symptoms.

In this study, too, the patients were not selected on the basis of the type of diabetes they were suffering from and the type of therapy they had received to control their hyperglycaemia and therefore in this case, too, it was impossible to understand what the cause of the improvement was and in what subgroup of patients it occurred.

The authors suggest that further studies should be carried out in larger patient groups.

As already mentioned, in this case, too, the authors were wrong: it was not by increasing the number of patients recruited into the clinical trial that the investigators would have been able to understand whether or not the study compound was active in the treatment of diabetic peripheral neuropathy; these objectives would have been achievable only through a different selection of the patients to be included in the clinical trial.

The results obtained in the clinical trials cited above, which at times are contradictory and at times present no apparent contradictions, show a number of improvements in the treated groups compared to the control groups. In these studies the patients were not selected on the basis of the type of diabetes or the type of therapy they had received up to the time of their entry into the clinical trial.

In other words, the authors of these studies did not pose the problem as to whether, within the patient population recruited there might be a group of responders and a group of non-responders because they were not aware that different patient selection would have led to better and less variable results.

The result of the lack of patient selection with different inclusion criteria from those adopted was that the authors were unable to understand the reason why there were improvements in certain cases and not in others. For this reason, the authors endowed with greater critical sense declared that it was only by increasing the number of patients and the duration of the studies that it would be possible to demonstrate (without any residual doubts) whether the study compound was active or not. The present invention demonstrates that these authors were wrong, because it was not by increasing the size of the study population or the length of the treatment periods, using the same inclusion criteria, that it would be possible to demonstrate that acetyl L-carnitine is active for the treatment of diabetic neuropathy in the patients recruited and treated. These objectives would be achievable only by adopting different inclusion criteria.

Previous uses of acetyl L-carnitine are already known.

For example, US Patent 4,751,242 describes the use of acetyl L-carnitine for the treatment of peripheral neuropathies, of various origins, including diabetic neuropathy.

In this patent, no information is provided regarding the type of diabetes or the type of treatment the patients had received to control their hyperglycaemia.

In this patent, there is no description or suggestion to the effect that a different patient inclusion criterion would have yielded better therapeutic results. In fact, a different selection of patients would have avoided non-responders being included in the study and treated with a compound which was of no use to them, i.e. ineffective, thus exposing these patients to the risk of incurring side effects without receiving any therapeutic benefit.

As already mentioned, in this case, thanks to the well-known lack of severe side effects of the study compound used, non-responders were not harmed by the drug, but the same could not be said if the study compound had been another drug, associated with more serious side effects.

Thus, in this study, too, patients were not selected on the basis of the type of therapy they had received to control their hyperglycaemia, and therefore it was impossible to understand in what group of treated patients the improvement occurred.

Further uses of acetyl L-carnitine are equally well known.

US Patent 5192805 relates to the use of acetyl L-carnitine in the therapeutic treatment of coma.

US 6,037,3721128 relates to the use of acetyl L-carnitine, isovaleryl L-carnitine and propionyl L-carnitine for increasing IGF-1 levels, for the treatment of lateral amyotrophic sclerosis, optic and olfactory nerve neuropathies, trigeminal nerve neuralgia and other pathologies.

US 6037372 relates to the use of an alkanoyl L-carnitine, including acetyl L-carnitine, for the treatment of diseases mediated by glutamate, such as epilepsy, schizophrenia, chronic fatigue syndrome, lateral amyotrophic sclerosis and other pathologies.

It has now been found that acetyl L-carnitine lends itself to use as an effective agent for the treatment of neuropathic pain in patients with type 2 diabetes not treated with insulin.

As already mentioned, the use of acetyl L-carnitine for the treatment of neuropathies is already known. What is still unknown today at the time of filing the present application is that acetyl L-carnitine is useful for treating diabetic neuropathies in patients with type 2 diabetes non on insulin treatment.

To date, in the clinical trials in which acetyl L-carnitine has been used for the treatment of peripheral neuropathy in diabetic patients, no distinction has been made between the various types of diabetes and the various types of pharmacological treatment the diabetic patients had received.

This lack of appropriate patient selection entailed the treating of patients who (as will be demonstrated in the experimental part here below) failed to respond to treatment with acetyl L-carnitine, and very variable experimental results were obtained which did not guarantee the complete efficacy of the study compound.

In fact, in Drugs 1997 Sept: 54 (3) 414-421 it is reported that:
1) the efficacy of acetyl L-carnitine is poor;
2) in the context of the drugs proposed for the treatment of peripheral neuropathy, to date (September 1997) there are no data or clinical trials justifying their use in diabetic patients;
3) a recent clinical trial conducted in Italy in approximately 200 patients with diabetic neuropathy has failed to show any significant difference between acetyl L-carnitine and placebo (Sigma-Tau Pharmaceuticals, unpublished data protocol No. DRN 2003289I/ALC-ST200);
4) as of today, there are no drugs useful for the treatment of diabetic neuropathy owing to (a) the poor methodological quality of most of the trials conducted to date; (b) insufficient trial duration; (c) methodological difficulties related to the patient inclusion and exclusion criteria;
5) future clinical trials will have to take these problems into account.

The expert in the field knows that from the date of publication of Drugs 1997 Sept. 54 (3) 414-421 to today there have been no changes in the patient inclusion criteria, and therefore the difficulties encountered in interpreting the results have remained the same.

Thanks to the present invention we now know that a different method of selecting the patients to be included in clinical trials, and therefore treated, would have demonstrated the efficacy of the study compound without leaving any residual doubts regarding the interpretation of the results (see the experimental part here below).

The subject of the present invention is therefore the use of acetyl L-carnitine, or one of its pharmaceutically acceptable salts, for the preparation of a medicine for the treatment of neuropathic pain in patients with type 2 diabetes not treated with insulin.

What is meant by pharmaceutically acceptable salt of acetyl L-carnitine is any salt of the latter with an acid that does not give rise to unwanted toxic or side effects. These acids are well known to experts in pharmaceutical technology.

Examples of such salts, though not exclusively the following, are: chloride, bromide, orotate, acid aspartate, acid citrate, magnesium citrate, acid phosphate, fumarate and acid fumarate, magnesium fumarate, lactate, maleate and acid maleate, mucate, acid oxalate, pamoate, acid pamoate, acid sulphate, glucose phosphate, tartrate, acid tartrate, magnesium tartrate, 2-amino-ethane sulphonate, magnesium 2-amino-ethane sulphonate, choline tartrate and trichloroacetate.

The following examples illustrate the invention.

### EXAMPLE 1

Two double-blind placebo-controlled clinical trials, identical in their experimental design, were conducted in patients with diagnoses of diabetic peripheral neuropathy selected according to the criteria established in the San Antonio, Texas conference *(*Diabetes 37:1000-1004, 1988).

Acetyl L-carnitine was administered orally at doses of 1.5 g/ day and 3.0 g/ day for continuously for 52 weeks.

One objective of the studies consisted in the possible modification of the Visual Analogue Scale (VAS) (Scott J. Huskisson EC. Graphic representation of pain. Pain 1976;2:175-184) in the subjective assessment of pain symptoms and the main sensorimotor clinical parameters.

Two populations of patients with type 2 diabetes were treated, the first of which comprised patients not treated with insulin; this population included the presence of a control group of patients who did not receive the study compound (placebo, Group 1) and who matched up to the same inclusion criteria as the patients treated with acetyl L-carnitine (not on insulin treatment).

The second population comprised patients with type 2 diabetes treated with insulin. Here, too, the population included a placebo group (Group 4) matching up to the same inclusion criteria as the patients treated with acetyl L-carnitine (on insulin treatment).

The overall results obtained in the patients of the two studies are presented in Tables 1 and 2.

**Table 1**

| Patients with type 2 diabetes not on insulin treatment | | | |
|---|---|---|---|
| | | **Results** | |
| **Group** | **Treatment** | **VAS** | **"*P* value" vs. placebo** |
| 1 | Placebo (n = 28 pts) | 3.4 ± 23.3 | - |
| 2 | Acetyl L-carnitine 1.5 g/day (n = 39 pts) | 7.6 ± 34.7 | *P* = N.S. |
| 3 | Acetyl L-carnitine 3.0 g/day (n=40 pts) | 24.2 ± 32.3 | *P* = 0-006 |

**Table 2**

| Patients with type 2 diabetes on insulin treatment | | | |
|---|---|---|---|
| | | **Results** | |
| **Group** | **Treatment** | **VAS** | **"*P* value" vs. placebo** |
| 4 | Placebo (n = 58 pts) | 14.2 ± 32.2 | - |
| 5 | Acetyl L-carnitine 1.5 g/ die (n = 41 pts) | 16.8 ± 30.1 | *P* = N.S. |
| 6 | Acetyl L-carnitine 3.0 g/day (n = 57 pts) | 25.9 ± 31.2 | *P* = N.S. |

The results reported in Table 1 show that, at the end of treatment in patients with type 2 diabetes not on insulin treatment, acetyl L-carnitine was active in a dose-related manner, i.e. only at the higher dose (3 g/day), compared to the placebo group.

In contrast, the results reported in Table 2 show that the same compound, used in patients with type 2 diabetes on insulin treatment, showed no statistically significant difference in activity, even at the higher dose, compared to the control group.

It is interesting to note in Table 2 that the placebo group and the patient group treated with the lower dose of acetyl L-carnitine (1.5 g/ day) present higher VAS scores, at the end of treatment, than the equivalent groups in Table 1; it is believed that this difference may be attributable to the effect of the insulin treatment, which is no longer detectable at the higher dose of the study compound.

If the study compound had also been active in the type 2 diabetic patients on insulin treatment, the difference in activity vs. the placebo group (Group 4) and the group receiving acetyl L-carnitine at the lower dose (Group 5) should have been maintained in that population.

The results presented in Tables 1 and 2 are the demonstration that the clinical trials carried out in the past, which did not envisage patient selection according to the principles outlined in the present invention, showed signs of activity which were not to be regarded as predictive of real activity in the entire population treated, but were simply casual positive responses, without the investigators understanding the real reason for them. These responses were caused in part by the real clinical improvement in a number of patients and by the placebo effect in the others.

Acetyl L-carnitine is a known compound, whose preparation procedure is described in US patents US 4439438 and US 4254053.

The acetyl L-carnitine can be in any form suitable for oral or parenteral administration in human subjects.

On the basis of various factors, such as the concentration of the active ingredient or the patient's condition, the compound according to the invention can be marketed as a food supplement or nutritional supplement, or as a therapeutic product on sale either with or without a compulsory doctor's prescription.

It has been found that, although the daily dose of the above-mentioned active ingredient to be administered depends on the patient's age, weight and general condition, using professional experience, it is generally advisable to administer, whether as a single dose or in multiple doses, approximately 1.5 to 5 g/day of acetyl L-carnitine, or an equimolar amount of one of its pharmaceutically acceptable salts. The preferred doses are greater than 1.5 g/day and may be as much as the maximum dose advised, thanks to the extremely low toxicity of said active ingredient.

The medicine according to the present invention can be prepared by mixing the active ingredient (acetyl L-carnitine inner salt or one of its pharmaceutically acceptable salts) with suitable excipients for the formulation of compositions for enteral administration (particularly oral administration) or for parenteral administration (particularly via the intramuscular o intravenous routes).

Experts in pharmaceutical technology are familiar with said excipients.

The pharmaceutically acceptable salts of the above-mentioned active ingredients include all the pharmaceutically acceptable salts which are prepared by addition of an acid to acetyl L-carnitine inner salt, and which do not give rise to unwanted toxic or side effects. The formation of salts by addition of an acid is well known in pharmaceutical technology.

## Claims

1. Use of acetyl L-carnitine, or one of its pharmaceutically acceptable salts, for the preparation of a medicine for the treatment of neuropathic pain in patients with type 2 diabetes not treated with insulin, in which acetyl L-carnitine is administered orally at a dose of 3 g/day.

2. Use according to claim 1, in which the pharmaceutically acceptable salt of acetyl L-carnitine is selected from the group consisting of chloride, bromide, orotate, acid aspartate, acid citrate, magnesium citrate, acid phosphate, fumarate and acid fumarate, magnesium fumarate, lactate, maleate and acid maleate, mucate, acid oxalate, pamoate, acid pamoate, acid sulphate, glucose phosphate, tartrate, acid tartrate, magnesium tartrate, 2-amino-ethane sulphonate, magnesium 2-amino-ethane sulphonate, choline tartrate and trichloro-acetate.

## Patentansprüche

1. Verwendung von Acetyl-L-Carnitin oder eines seiner pharmazeutisch unbedenklichen Salze zur Herstellung eines Arzneimittels zur Behandlung neuropathischer Schmerzen bei Patienten mit Typ-2-Diabetes, der nicht mit Insulin behandelt wird, wobei Acetyl-L-Carnitin oral in einer Dosis von 3 g/Tag verabreicht wird.

2. Verwendung nach Anspruch 1, wobei das pharmazeutisch unbedenkliche Salz von Acetyl-L-Carnitin aus der Gruppe bestehend aus Chlorid, Bromid, Orotat, Säureaspartat, Säurecitrat, Magnesiumcitrat, Säurephosphat, Fumarat und Säurefumarat, Magnesiumfumarat, Lactat, Maleat und Säuremaleat, Mucat, Säureoxalat, Pamoat, Säurepamoat, Säuresulfat, Glucosephosphat, Tartrat, Säuretartrat, Magnesiumtartrat, 2-Aminoethansulfonat, Magnesium-2-aminoethansulfonat, Cholintartrat und Trichloracetat ausgewählt ist.

## Revendications

1. Utilisation de l'acétyl L-carnitine, ou de l'un de ses sels pharmaceutiquement acceptables, pour la préparation d'un médicament pour le traitement de la douleur neuropathique chez des patients atteints du diabète de type 2 non traités par de l'insuline, dans laquelle l'acétyl L-carnitine est administrée par voie orale à une dose de 3 g/jour.

2. Utilisation selon la revendication 1, dans laquelle le sel pharmaceutiquement acceptable de l'acétyl L-carnitine est choisi dans le groupe consistant en chlorure, bromure, orotate, aspartate acide, citrate acide, citrate de magnésium, phosphate acide, fumarate et fumarate acide, fumarate de magnésium, lactate, maléate et maléate acide, mucate, oxalate acide, pamoate, pamoate acide, sulfate acide, phosphate de glucose, tartrate, tartrate acide, tartrate de magnésium, 2-amino-éthane sulfonate, 2-amino-éthane sulfonate de magnésium, tartrate de choline et trichloro-acétate.
